(19) Europäisches Patentamt
European Patent Office
Office européen des brevets

(11) Publication number: 0 015 758
B1

(12) # EUROPEAN PATENT SPECIFICATION

(45) Date of publication of patent specification: 05.05.82

(51) Int. Cl.$^3$: C 07 C 15/46, C 07 C 4/00, B 01 J 29/04

(21) Application number: 80300696.4

(22) Date of filing: 06.03.80

(54) **Process for producing styrenes.**

(30) Priority: 08.03.79 JP 27437/79
14.08.79 JP 103657/79

(43) Date of publication of application:
17.09.80 Bulletin 80/19

(45) Publication of the grant of the patent:
05.05.82 Bulletin 82/18

(84) Designated Contracting States:
CH DE FR GB IT NL

(56) References cited:
US - A - 2 420 689
US - A - 2 422 163

(73) Proprietor: SUMITOMO CHEMICAL COMPANY, LIMITED
15 Kitahama 5-chome Higashi-ku
Osaka-shi Osaka-fu (JP)

(72) Inventor: Matsuda, Teruo
7-33, Maedacho
Niihama-shi Ehime (JP)
Inventor: Shirafuji, Tamio
2-215, Ikkucho 2-chome
Niihama-shi (JP)
Inventor: Beppu, Masazo
20-1 Hoshigoecho
Niihama-shi Ehime (JP)
Inventor: Shimizu, Toyomitzu
9-16, Maedacho
Niihama-shi Ehime (JP)

(74) Representative: Myerscough, Philip Boyd et al,
J.A.Kemp & Co. 14, South Square Gray's Inn
London, WC1R 5EU (GB)

EP 0 015 758 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for producing styrenes

The present invention relates to a process for producing styrene or nucleus-substituted styrenes by the catalytic decomposition of 1,1-diarylethanes in gaseous phase.

Styrene and nucleus-substituted styrenes are very useful as intermediates for production of plastics and fine chemicals such as agricultural chemicals.

It is well known that the styrene and nucleus-substituted styrenes are produced by catalytically decomposing 1,1-diarylethanes at 350°C to 600°C in the presence of a solid catalyst such as silica, alumina, silica-alumina or kaolin (J. K. Dixon and K. W. Saunders: I. E. C., *46*, No. 4, 652), or by decomposing 1,1-diarylethanes at 350°C to 600°C in the presence of silica gel with continuous addition of hydrogen chloride gas or its aqueous solution (Published Examined Japanese Patent Application No. 28499/1974).

As a result of the examination of these well-known methods, however, the followings were found by the present inventors: in carrying out the decomposition of 1,1-ditolylethane using silica-alumina as catalyst, when a conversion of 1,1-ditolylethane is 60 to 80%, the selectivity of p-methylstyrene is as low as 40 to 60%, and p-ethyltoluene which is a hydrogenated product of p-methylstyrene, is produced as by-product in an amount as much as 30 to 40%; and in carrying out the decomposition of 1,1-ditolylethane using silica gel and hydrogen chloride in combination, when a conversion of 1,1-ditolylethane is 50 to 70%, the selectivity of p-methylstyrene is 235 to 35% and the amount of ethyltoluene, a by-product, is as low as 3% but considerable amounts of p-methylstyrene polymer are produced.

The present inventors extensively studied to improve these well-known methods, and found that zeolite is very superior as a catalyst for decomposition of 1,1-diarylethane.

The present invention is to provide a process for producing a styrene of the formula (II) and/or (III)

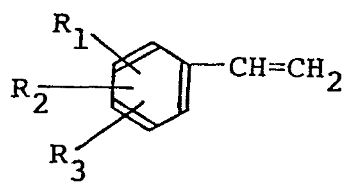

(II)

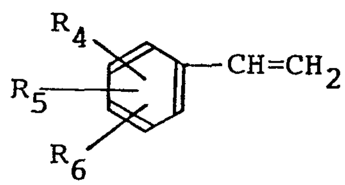

(III)

wherein $R_1$ to $R_6$ are each a hydrogen atom or a methyl group, which comprises subjecting a 1,1-diarylethane of the formula (I),

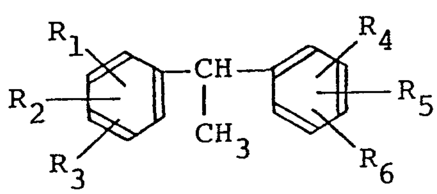

(I)

wherein $R_1$ to $R_6$ are as defined above, to gas-phase catalytic decomposition at a temperature of 300°C to 700°C in the presence of zeolite as a catalyst.

The present invention will be illustrated in more detail hereinafter.

1,1-Diarylethanes used as a starting material in the present invention include for example 1,1-diphenylethane, 1-phenyl-1-tolylethane, 1-phenyl-1-xylylethane, 1-phenyl-1-trimethylphenylethane, 1,1-ditolylethane, 1-tolyl-1-xylylethane, 1-tolyl-1-trimethylphenylethane, 1,1-dixylylethane, 1-xylyl-1-trimethylphenylethane and 1,1-bis(trimethylphenyl)ethane. Of these, 1,1-ditolylethane and 1,1-dixylylethane are particularly effectively applied for the catalytic decomposition in accordance with the present invention.

The 1,1-diarylethane may be synthesized by any method. For example, it can be synthesized by reacting an aromatic compound with acetaldehyde or styrenes at low temperatures with sulfuric acid as catalyst, or by reacting an aromatic compound with acetylene with mercury sulfate and conc. sulfuric acid as catalyst.

Preferred zeolite used as the catalyst in the present invention is such that the diameter of pores is 5 to 15 Å and the molar ratio of $SiO_2$ to $Al_2O_3$ is 0.5 to 400:1, particularly preferably 1 to 300:1. When the molar ratio of $SiO_2$ to $Al_2O_3$ is below this range, such zeolite is unsuitable as the catalyst because of its poor heat resistance. While when the molar ratio is far beyond this range, the development of acidity is insufficient so that the catalytic activity is lowered. Zeolite used in the present invention may be any of the H-form (de-cation form), alkaline earth metal-substituted form and rare earth metal-substituted form. Also, zeolite used in the present invention may be natural zeolite or synthetic one, and includes, for example, the X-form and the Y-form of faujasite, mordenite, chabazite and the like.

In the present invention, zeolite used as the catalyst can be dealuminum-treated in advance with an inorganic or organic acid to increase the molar ratio of silica/alumina as well as the surface area and pore volume of the catalyst, whereby the conversion of 1,1-diarylethane and the selectivity of styrenes can remarkably be improved. Any zeolite may be used for the preparation of dealuminum-treated zeolite so far as it has a pore diameter of 5 to 15 Å, a $SiO_2/Al_2O_3$ molar ratio of 2.5 or more and a strong acid resistance. Example of such starting zeolite include super-stable zeolite produced by cation-exchanging the Y-form faujasite with an ammonium ion, followed by baking, synthetic mordenite and L-form zeolite.

In carrying out the dealuminum-treatment, such zeolite is subjected to heating under reflux with an inorganic or organic acid so as to make the molar ratio of $SiO_2$ to $Al_2O_3$ 5 to 400, particularly preferably 10 to 300.

In order to effectively attain the desired molar ratio, it is preferred to repeat several times a manner including the aforesaid heat-treatment, separation of the treated zeolite and then drying. In this manner, the drying includes a baking at 300° to 700°C in the stream of steam, nitrogen or air or a mixture thereof.

As the acid used for preparing the catalyst, dealuminum-treated zeolite, of the present invention, any of inorganic acids and organic ones, for example hydrochloric acid, sulfuric acid, nitric acid and acetic acid, may be used. Particularly, however, hydrochloric acid is preferred. The concentration of the acid is 0.2 to 10 N, particularly preferably 0.5 to 5 N. When the acid concentration is below this range, a time required for the $SiO_2/Al_2O_3$ molar ratio to reach the pre-determined value becomes too long. While when the concentration is far beyond this range, the crystallinity of zeolite becomes extremely poor so that zeolite becomes similar to non-crystalline silica-alumina. As a result, the amount of ethyltoluene, a by-product, increases and the selectivity of methylstyrene becomes low.

In carrying out the catalytic decomposition, the reaction temperature is controlled to 300° to 700°C, particularly preferably 400° to 600°C. Reaction temperatures far beyond this range promotes side reactions to lower the selectivity. While reaction temperatures far below this range lower the reaction rate to result in economical disadvantage.

The reaction pressure is not particularly limited so far as the reaction system maintains a gaseous phase under the reaction conditions. Generally, however, it is preferred to carry out the reaction under atmospheric or increased pressures.

In the present invention, the reaction system may contain one or more of nitrogen gas, carbon dioxide gas, steam and hydrogen gas in addition to 1,1-diarylethane, a starting material. The amount of the gas added is preferably 0.005 to 50 moles per mole of the 1,1-diarylethane. When these gases are not added to the reaction system, the deposition of carbon onto catalyst becomes so remarkable that the life of catalyst is shortened. Amounts of the gas beyond the above range are not particularly unfavorable to the reaction, but they are not desirable on account of economical disadvantages.

Particularly when the reaction is carried out in a reductive atmosphere containing hydrogen gas, the amounts of hydrogenated by-products of styrene or nucleus-substituted styrenes (e.g. ethylbenzene, ethyltoluene, ethylxylene, ethyltrimethylbenzene) are decreased to result in an increase in the conversion of 1,1-diarylethane and selectivity of styrene or nucleus-substituted styrenes as well as in the remarkable prolongation of the life of catalyst.

The amount of hydrogen is preferably 0.01 to 50 moles, more preferably 0.1 to 20 moles, per mole of the 1,1-diarylethane. The aforesaid additive effect cannot be attained in amounts of hydrogen below this range. Amounts of hydrogen beyond this range are not particularly unfavorable to the reaction, but they are not undesirable on account of economical disadvantages.

In the present invention, the space velocity of the reaction gas is preferably 500 to 20,000 $hr^{-1}$, particularly preferably 1,000 to 12,000 $hr^{-1}$. But the space velocity may optionally be selected according to the composition of reaction gas, kind of catalyst and reaction temperature.

The reaction of the present invention may be carrued out by any process of a fixed-bed, moving-bed or fluidized-bed process.

The present invention will be illustrated in more detail with reference to the following examples, which are not however to be interpreted as limiting the invention thereto. Unless otherwise stated, all percents in the examples are by mole.

Examples 1 to 5 and Comparative Example 1

Five milliliters of the 10- to 16-mesh catalyst shown in Table 1 was placed in the central portion of a quartz tube reactor (length 300 mm, internal diameter 10 mm), and silicon carbide as a pre-heater

was packed above the catalyst. The catalyst layer was then heated to 500°C.

Reaction was carried out continuously for 8 hours while supplying 5 g (24 mmole)/hr of 1,1-ditolylethane, 8.6 g (480 mmole)/hr of steam and 3.8 liter (170 mmole/hr) of nitrogen gas. The mixed gas from the reactor was liquefied by a cooler, and the organic layer was analyzed by gas chromatography. The results are shown in Table 1.

TABLE 1

| | Catalyst | Conversion of ditolylethane (%) | Selectivity of methylstyrene (%) | Selectivity of ethyltoluene (%) |
|---|---|---|---|---|
| Example 1 | H-substituted mordenite * | 93 | 89 | 0.04 |
| Example 2 | H-substituted X-form faujasite | 85 | 87 | 0.7 |
| Example 3 | H-substituted Y-form faujasite | 91 | 88 | 0.5 |
| Example 4 | La-substituted Y-form faujasite | 90 | 87 | 0.5 |
| Example 5 | 10 X (Ca-form) ** | 82 | 85 | 0.9 |
| Comparative Example 1 | silica-alumina *** | 65 | 63 | 28 |

* Zeolon H-form (produced by Norton Co.)

** 10 X (Ca-form) (produced by W. R. Grace & Co.)

*** N—631—L (produced by Nikki Kagaku Co.)

Example 6

Reaction was carried out in the same manner as in Example 1 except that 5.7 g (24 mmole)/hr of 1,1-dixylylethane was used in place of 1,1-ditolylethane. As a result, it was found that the conversion of dixylylethane was 90% and the selectivities of dimethylstyrene and ethylxylene were 90% and 0.1%, respectively.

Examples 7 to 9 and Comparative Example 2

Ten milliliters of the catalyst of a cylindrical form (2 mmø x 2 mmH) shown in Table 2 was placed in the central portion of a quartz tube reactor (length 300 mm, internal diameter 10 mm), and silicon carbide as a pre-heater was packed above the catalyst. The catalyst layer was then heated to 500°C.

Reaction was carried out continuously for 5 hours while supplying 37.8 g (0.18 mole)/hr of 1,1-ditolylethane, 16.2 g (0.90 mole)/hr of steam and 4.02 liter (0.18 mole)/hr of hydrogen. The mixed gas from the reactor was liquefied by a cooler, and the organic layer was analyzed by gas chromatography. The results are shown in Table 2.

TABLE 2

| | Catalyst | Time elapsed after the beginning of reaction (hr) | Conversion of ditolylethane (%) | Selectivity of methylstyrene (%) | Selectivity of ethyltoluene (%) |
|---|---|---|---|---|---|
| Example 7 | H-substituted mordenite * | 1<br>5 | 50<br>40 | 94<br>93 | 4<br>5 |
| Example 8 | H-substituted X-form faujasite | 1<br>5 | 50<br>36 | 89<br>87 | 6<br>8 |
| Example 9 | H-substituted Y-form faujasite | 1<br>5 | 55<br>40 | 92<br>90 | 5<br>6 |
| Comparative Example 2 | silica-alumina** | 1<br>5 | 50<br>30 | 70<br>65 | 25<br>26 |

* Zeolon H-form (produced by Norton Co.)

** N–631–L (produced by Nikki Kagaku Co.)

Examples 10 to 12 and Comparative Example 3

Reaction was carried out in the same manner as in Examples 7 to 9 and Comparative Example 2 except that 4.02 liter/hr of nitrogen was used in place of hydrogen. The results are shown in Table 3.

TABLE 3

| | Catalyst | Time elapsed after the beginning of reaction (hr) | Conversion of ditolylethane (%) | Selectivity of methylstyrene (%) | Selectivity of ethyltoluene (%) |
|---|---|---|---|---|---|
| Example 10 | H-substituted mordenite * | 1<br>5 | 32<br>15 | 87<br>86 | 8<br>9 |
| Example 11 | H-substituted X-form faujasite | 1<br>5 | 35<br>17 | 90<br>80 | 5<br>13 |
| Example 12 | H-substituted Y-form faujasite | 1<br>5 | 35<br>17 | 85<br>80 | 8<br>12 |
| Comparative Example 3 | silica-alumina** | 1<br>5 | 30<br>10 | 64<br>50 | 30<br>35 |

* Zeolon H-form (produced by Norton Co.)

** N–631–L (produced by Nikki Kagaku Co.)

Example 13

Reaction was carried out in the same manner as in Example 7 except that 42.8 g (0.18 mole)/hr of 1,1-dixylylethane was used in place of 1,1-ditolylethane. The result is shown in Table 4.

TABLE 4

| | Time elapsed after the beginning of reaction (hr) | Conversion of dixylylethane (%) | Selectivity of dimethylstyrene (%) | Selectivity of ethylxylene (%) |
|---|---|---|---|---|
| Example 13 | 1<br>5 | 53<br>42 | 90<br>89 | 5<br>7 |

Examples 14 to 18

The catalyst used in Example 14 was prepared as follows: Forty grams of H-form mordenite (trade name, Zeolon 900H) of a cylindrical form (2 mmø x 2 mmH) was dipped in 920 ml of 2N hydrochloric acid, heated to 100°C for 4 hours and filtered. This acid treatment was repeated four times. The treated mordenite was then washed with ion-exchange water until chlorine ions were no longer detectable. After drying, the mordenite was baked at 500°C for 5 hours in air. The molar ratio of silica to alumina was 20.2.

The catalyst used in Example 15 was prepared as follows: Forty grams of H-form mordenite (trade name, Zeolon 900H) of a cylindrical form (2 mmø x 2 mmH) was baked at 540°C for 2 hours in the stream of steam (steam 10 g/hr, nitrogen 3 liter/hr). Thereafter, the baked mordenite was dipped in 920 ml of 2N hydrochloric acid and heated to 100°C for 4 hours. The mordenite was filtered and washed with ion-exchange water until chlorine ions were no longer detectable. This cycle of baking and acid treatment was repeated four times, and then the mordenite was baked at 500°C for 4 hours in air. The molar ratio of silica to alumina was 43.4.

The catalyst used in Example 16 was prepared as follows: Procedure was carried out in the same manner as in Example 15 except that 3 liter/hr of nitrogen alone was passed in place of steam. The molar ratio of silica to alumina was 32.8.

The catalyst used in Example 17 was prepared as follows: One hundred grams of Na-substituted Y-form faujasite (trade name, SK-40, produced by Linde Co.) was baked at 370°C for 3 hours. Thereafter, it was treated in the following order: It was placed in a mixture of water (750 g) and ammonium sulfate (150 g), stirred at room temperature for 1 hour and filtered; treated at 100°C for 2 hours in a mixture of water (750 g) and ammonium sulfate (150 g), filtered and washed with water until sulfate ions were no longer detectable; baked at 540°C for 2 hours; treated at 100°C for 3 hours in a mixture of water (6,000 g) and ammonium sulfate (300 g), and filtered; treated at 100°C for 1 hour in a mixture of water (6,000 g) and ammonium sulfate (300 g), filtered and washed with water until sulfate ions were no longer detectable; baked at 820°C for 3 hours. Forty grams of the H-substituted Y-form faujasite thus obtained was further treated in the following order: It was baked at 540°C for 2 hours in the stream of nitrogen (3 liter/hr); dipped in 920 ml of 2N hydrochloric acid, heated to 100°C for 4 hours, filtered and washed with ion-exchange water until chlorine ions were no longer detectable; and thereafter baked at 500°C for 4 hours in air. The $SiO_2/Al_2O_3$ molar ratio of this catalyst was 13.3.

The catalyst used in Example 18 was prepared as follows: Forty grams of K-substituted L-form zeolite (SK-45, produced by Linde Co.) was placed in 200 cc of an ammonium chloride-saturated liquor and refluxed for 5 hours. This operation was repeated further twice. The zeolite was then washed with water until chlorine ions were no longer detectable and baked at 500°C for 3 hours. The zeolite was further baked at 540°C for 2 hours in the stream of nitrogen (3 liter/hr), cooled, dipped in 920 ml of 2N hydrochloric acid, heated to 100°C for 4 hours, filtered and then washed with ion-exchange water until chlorine ions were no longer detectable. This cycle of baking in nitrogen stream, acid treatment and washing with water was repeated four times. Thereafter, the zeolite was dried and baked at 500°C for 4 hours in air. The silica:alumina molar ratio of this catalyst was 112.2.

Ten milliliters of each catalyst thus obtained was placed in the central portion of a quartz tube reactor (length 300 mm, internal diameter 10 mm), and silicon carbide as a pre-heater was packed above the catalyst. The catalyst layer was then heated to 500°C.

Reaction was carried out continuously for 5 hours while supplying 37.8 g (0.18 mole)/hr of 1,1-ditolylethane, 16.2 g (0.90 mole)/hr of steam and 4.02 liter (0.18 mole)/hr of nitrogen. The mixed gas from the reactor was liquefied by cooling, and the organic layer was analyzed by gas chromatography. The results are shown in Table 5.

TABLE 5

| | Time elapsed after the beginning of reaction (hr) | Conversion of ditolylethane (%) | Selectivity of methylstyrene (%) | Selectivity of ethyltoluene (%) |
|---|---|---|---|---|
| Example 14 | 1<br>5 | 56<br>40 | 94<br>92 | 3<br>5 |
| Example 15 | 1<br>5 | 65<br>52 | 90<br>87 | 7<br>9 |
| Example 16 | 1<br>5 | 65<br>52 | 93<br>90 | 3<br>5 |
| Example 17 | 1<br>5 | 60<br>45 | 90<br>87 | 5<br>7 |
| Example 18 | 1<br>5 | 65<br>50 | 87<br>85 | 3<br>7 |

Examples 19 to 23

Reaction was carried out in the same manner as in Examples 14 to 18 except that 4.02 liter (0.18 mole)/hr of hydrogen was used in place of nitrogen. The results are shown in Table 6.

TABLE 6

| | Time elapsed after the beginning of reaction (hr) | Conversion of ditolylethane (%) | Selectivity of methylstyrene (%) | Selectivity of ethyltoluene (%) |
|---|---|---|---|---|
| Example 19 | 1<br>5 | 65<br>51 | 94<br>92 | 3<br>4 |
| Example 20 | 1<br>5 | 75<br>63 | 92<br>89 | 4<br>7 |
| Example 21 | 1<br>5 | 76<br>65 | 93<br>92 | 3<br>4 |
| Example 22 | 1<br>5 | 69<br>54 | 91<br>88 | 4<br>6 |
| Example 23 | 1<br>5 | 73<br>59 | 90<br>88 | 2<br>5 |

Examples 24 and 25

Reaction was carried out in the same manner as in Examples 16 and 21 except that 42.8 g (0.18 mole)/hr of 1,1-dixylylethane was used in place of 1,1-ditolylethane. The results are shown in Table 7.

TABLE 7

| | Time elapsed after the beginning of reaction (hr) | Conversion of dixylylethane (%) | Selectivity of dimethylstyrene (%) | Selectivity of ethylxylene (%) |
|---|---|---|---|---|
| Example 24 | 1<br>5 | 67<br>53 | 92<br>90 | 4<br>5 |
| Example 25 | 1<br>5 | 75<br>62 | 93<br>92 | 3<br>4 |

## Claims

1. A process for producing a styrene of the formula (II) and/or (III),

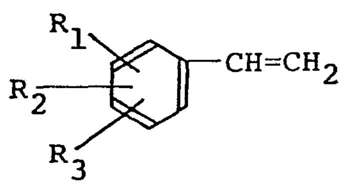
(II)

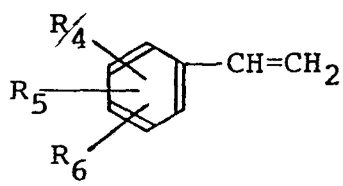
(III)

wherein $R_1$ to $R_6$ are each a hydrogen atom or a methyl group, by the gas-phase catalytic decomposition at an elevated temperature of a 1,1-diarylethane of the formula (I),

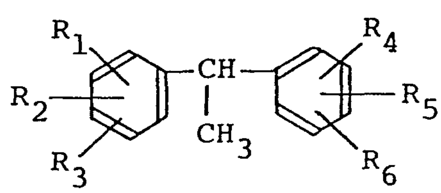
(I)

wherein $R_1$ to $R_6$ are as defined above, characterised in that the gas-phase catalytic decomposition is carried out at a temperature of 300°C to 700°C in the presence of zeolite as a catalyst.

2. A process according to claim 1, characterised in that said gas-phase catalytic decomposition is carried out in an atmosphere of a gas selected from hydrogen, nitrogen, carbon dioxide and steam and mixtures containing at least two of them.

3. A process according to claim 2, characterised in that the gas is used in an amount of 0.005 to 50 moles per mole of 1,1-diarylethane.

4. A process according to claim 1, 2 or 3, characterised in that the zeolite is of an H-form (decation form), alkaline earth metal-substituted form, or rare earth metal-substituted form.

5. A process according to any one of claims 1 to 4, characterised in that the zeolite is faujasite, mordenite or chabazite.

6. A process according to any one of claims 1 to 5, characterised in that the zeolite has pores 5 to 15Å in diameter and an $SiO_2/Al_2O_3$ molar ratio of 0.5 to 400:1.

7. A process according to any one of claims 1 to 6, characterised in that the zeolite has been dealuminum-treated with an inorganic or organic acid.

8. A process according to claim 7, characterised in that the $SiO_2/Al_2O_3$ molar ratio of the dealuminum-treated zeolite is 5 to 400:1.

9. A process according to any one of claims 1 to 8, characterised in that the gas-phase catalytic decomposition is carried out at a space velocity of 500 to 20,000 $hr^{-1}$.

**Revendications**

1. Procédé de production d'un styrène de formule (II) et/ou (III)

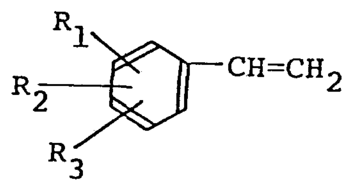

(II)

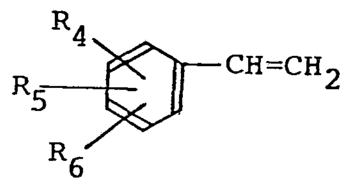

(III)

où les substituants $R_1$ à $R_6$ sont chacun un atome d'hydrogène ou un groupement méthyle, par décomposition catalytique en phase gazeuse, à température élevée, d'un 1,1-diaryléthane de formule (I)

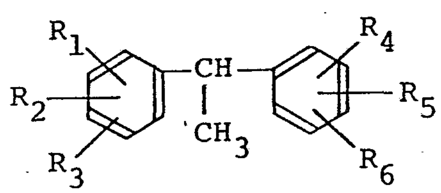

(I)

où les substituants $R_1$ à $R_6$ sont tels que définis précédemment, caractérisé en ce qu'on effectue la décomposition catalytique en phase gazeuse à une température de 300°C à 700°C en présence de zéolite comme catalyseur.

2. Procédé selon la revendication 1, caractérisé en ce que ladite décomposition catalytique en phase gazeuse est effectuée dans une atmosphère d'un gaz choisi entre l'hydrogène, l'azote, le gaz carbonique et la vapeur d'eau et les mélanges contenant au moins deux d'entre eux.

3. Procédé selon la revendication 2, caractérisé en ce que le gaz et utilisé à raison de 0,005 à 50 moles par mole de 1,1-diaryléthane.

4. Procédé selon l'une quelconque des revendications 1, 2 ou 3, caractérisé en ce que la zéolite est sous forme H (forme décationisée), sous forme substituée par un métal alcalino-terreux ou sous forme substituée par une terre rare.

5. Procédé selon l'une quelconque des revendications 1 à 4, caractérisé en ce que la zéolite est la faujasite, la mordénite ou la chabazite.

6. Procédé selon l'une quelconque des revendications 1 à 5, caractérisé en ce que la zéolite a des pores d'un diamètre de 5 à 15 Å et un rapport molaire $SiO_2/Al_2O_3$ de 0,5 à 400:1.

7. Procédé selon l'une quelconque des revendications 1 à 6, caractérisé en ce que la zéolite a été traitée pour enlever de l'aluminium à l'aide d'un acide minéral ou organique.

8. Procédé selon la revendication 7, caractérisé en ce que le rapport molaire $SiO_2/Al_2O_3$ de la zéolite traitée pour enlever de l'aluminium est 5 à 400:1.

9. Procédé selon l'une quelconque des revendications 1 à 8, caractérisé en ce que la décomposition catalytique en phase gazeuse est effectuée à une vitesse spatiale de 500 à 20 000 $h^{-1}$.

**Patentansprüche**

1. Verfahren zur Herstellung eines Styrols der Formel (II) und/oder (III),

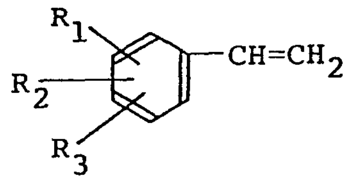

(II)

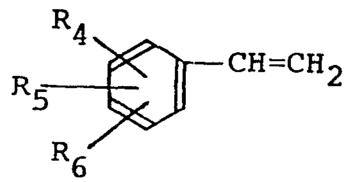

(III)

in welchen $R_1$ bis $R_6$ jeweils ein Wasserstoffatom oder einen Methylrest bedeuten, durch katalytische Zersetzung in der Gasphase bei einer erhöhten Temperatur eines 1,1-Diaryläthans der Formel (I)

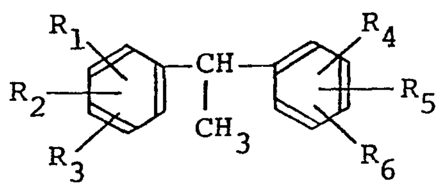

(I)

in welcher $R_1$ bis $R_6$ die obige Bedeutung haben, dadurch gekennzeichnet, daß die katalytische Zersetzung in der Gasphase bei einer Temperatur von 300°C bis 700°C in Anwesenheit von Zeolith als Katalysator durchgeführt wird.

2. Verfahren nach Anspruch 1, dadurch gekennzeichnet, daß die genannte katalytische Zersetzung in der Gasphase in einer Atmosphäre eines Gases durchgeführt wird, das ausgewählt ist unter Wasserdampf, Stickstoff, Kohlendioxid und Wasserdampf und Mischungen, die wenigstens zwei von diesen Stoffen enthalten.

3. Verfahren nach Anspruch 2, dadurch gekennzeichnet, daß das Gas in einer Menge von 0,005 bis 50 Mol pro Mol 1,1-Diaryläthan verwendet wird.

4. Verfahren nach Anspruch 1, 2 oder 3, dadurch gekennzeichnet, daß der Zeolith eine H-Form (De-Kation-Form), Erdalkalimetall-ausgetauschte Form oder Seltenes Erd-metall-ausgetauschte Form aufweist.

5. Verfahren nach irgendeinem der Ansprüche 1 bis 4, dadurch gekennzeichnet, daß der Zeolith Faujasit, Mordenit oder Chabasit ist.

6. Verfahren nach irgendeinem der Ansprüche 1 bis 5, dadurch gekennzeichnet, daß der Zeolith Poren mit einem Durchmesser von 5 bis 15 Å und ein $SiO_2/Al_2O_3$-Molverhältnis von 0,5 bis 400:1 aufweist.

7. Verfahren nach irgendeinem der Ansprüche 1 bis 6, dadurch gekennzeichnet, daß der Zeolith einer Behandlung mit einer anorganischen oder organischen Säure zum Entzug von Aluminium unterzogen worden ist.

8. Verfahren nach Anspruch 7, dadurch gekennzeichnet, daß das $SiO_2/Al_2O_3$-Molverhältnis des der Behandlung zum Entzug von Aluminium unterzogenen Zeoliths 5 bis 400:1 beträgt.

9. Verfahren nach irgendeinem der Ansprüche 1 bis 8, dadurch gekennzeichnet, daß die katalytische Zersetzung in der Gasphase bei einer Raumgeschwindigkeit von 500 bis 20.000 Stunden$^{-1}$ durchgeführt wird.